# EUROPEAN PATENT APPLICATION

(11) **EP 1 548 119 A1**
(43) Date of publication of application: **29.06.2005**
(21) Application number: 03798509.0
(22) Date of filing: 25.09.2003
(51) Int. Cl.: C12N 15/866, C12N 7/00, A61K 48/00

(54) **BACULOVIRUS VECTOR, METHOD OF CONSTRUCTING BACULOVIRUS VECTOR AND GENE TRANSFER METHOD**

(30) Priority: 25.09.2002 JP 2002278469
(71) Applicant: Osaka Industrial Promotion Organization, Osaka-shi, Osaka 540-0029 (JP)
(72) Inventor: MATSUURA, Yoshiharu, Suita-shi, Osaka 565-0821 (JP)
(74) Representative: Daniels, Jeffrey Nicholas
(86) International application number: PCT/JP2003/012275
(87) International publication number: WO 2004/029259

(57) **Abstract**

The present invention provides a novel baculovirus vector which can provide a desired protein not requiring infectivity to insect cells, on the viral particle surface; method of producing thereof; and method of gene transfer using the baculovirus vector. A method of producing a baculovirus vector including; a process of cotransfecting at least a plasmid containing a gene coding a protein capable of being expressed on a cell surface and one of wild-type, mutated-type form, and recombinant baculovirus DNAs into insect cells, wherein a pseudotyped baculovirus which includes at least one part of the baculovirus DNA and is coated with the protein capable of being expressed on a cell surface, is generated.

SEQUENCE LISTING

<110> Osaka Industrial Promotion Organization

<120> baculovirus vectors

<130> N-OS002-03P osaka TLO

<140>
<141>

<150>2002-278469
<151>2002-09-25

<160> 2

<170> PatentIn Ver. 2.1

<210> 1
<211> 30
<212> DNA
<213> Artificial Sequence

<220>
<223> Description of Artificial Sequence: primer for
gp64

<400> 1

aaagatctac catggtaagc gctattgttt                                    30

<210> 2
<211> 30
<212> DNA
<213> Artificial Sequence

<220>
<223> Description of Artificial Sequence: primer for
      gp64

<400> 2
ttgtcgactt aatattgtct attacggttt                                    30

aaagatctac catggtaagc gctattgttt 30

<210> 2

<211> 30

<212> DNA

<213> Artificial Sequence

<220>

<223> Description of Artificial Sequence: primer for

gp64

<400> 2

ttgtcgactt aatattgtct attacggttt 30

## Description

### Technical Field

The present invention relates to a baculovirus vector which can be suitably used for gene therapy, etc., a method of producing thereof and a method of gene transfer using the baculovirus vector.

### Background Art

Ten years have passed since clinical study of gene therapy started. Until now, satisfactory outcome is not necessarily achieved; however, this therapy, without doubt, will be at the center of the advanced medical treatment in this century. Whether or not the gene therapy will successfully advance depends on the development of gene transfer vector which enables introduction of genes into target cells safely and efficiently and has large integrate capacity.

Retrovirus, adenovirus, adeno-associated virus, etc. have been mainly used as the gene therapy vector. However, there were problems about safety such as the emergence of self-propagating virus and the activation of cancer gene due to random integration of genes, and satisfactory gene transfer efficiency and improvement of specific gene transfer method were not achieved. Further, there were problems such as cytotoxicity, induction of immune reaction, inactivation due to neutralization antibody.

In contrast, baculovirus, an insect virus, had been considered to infect insets alone until recently and had been utilized as a large-scale expression system of transgene using only insect cells.

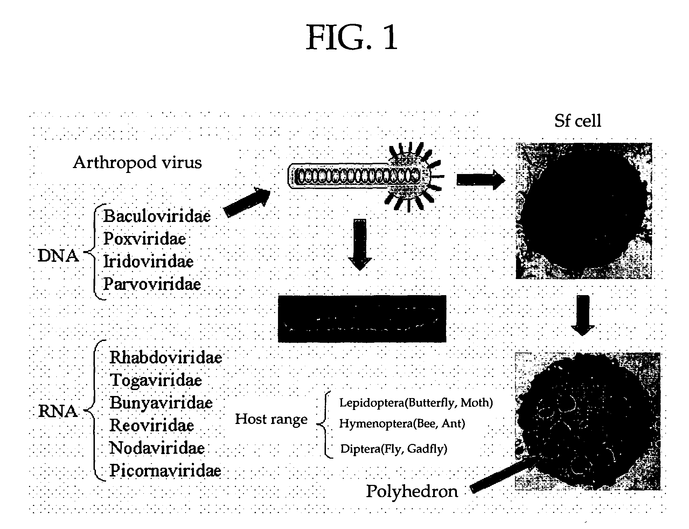

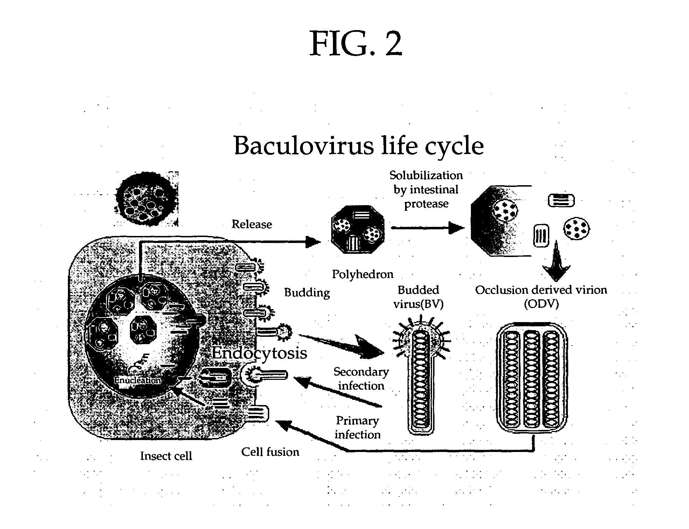

Baculoviruses are entomopathogenic viruses which infect the insects such as lepidopteran, hymenopteran and dipteran insect and have circular double-stranded DNA. Of the viruses, a group of viruses called Nuclear Polyhedorosis Virus (NPV) produce a large amount of occlusion body called polyhedron in the nucleus of the infected cell at late phase of infection. The amount of the occlusion body reaches as much as 40 to 50% of the total cell protein and the inclusion body includes a large number of viral particle (FIG. 1). Thus, even if the host is killed by infection, viruses in this body are protected from inactivation action from outside such as ultraviolet ray and heat, thus enabling the viruses to retain infectivity for a long time (FIG. 2).

Thus, polyhedron is essential for viruses to survive in nature, but it is not required for propagation of viruses. Therefore, even if a foreign gene to be expressed is introduced instead of a polyhedron gene, viruses infect, propagate and produce large amount of foreign gene products without any difficulties. Until now, two viruses, Autographa californica NPV (AcNPV) and Bombyx mori NPV (BmNPV) of silkworm, are used for vector, but in most cases, expression is carried out using AcNPV.

The present inventors have succeeded in the development of vector pAcYM1 which has the highest expression efficiency in the world among baculovirus vectors (e.g. refer to Non-patent document 1). They made the hepatitis C virus structural protein expressed in the system using the vector and succeeded in the development of early stage antibody diagnosis system using it as an antigen. Due to introduction of this diagnostic system, incidence of post-tranfusion hepatitis C is almost conquered in our country.

In recent years, gene transfer into mammalian cells using baculovirus was reported (e.g. refer to Non-patent documents 2 and 3). At first, it was regarded the gene transfer using bac ulovirus as specific to hepatocyte; however, the present invento rs revealed the capability of gene transfer into a wide variety of animal cells (e.g. refer to Non-patent document 4).

In this way, it has been revealed that baculovirus vectors infect a wide variety of mammalian cells and can express foreign genes efficiently without replication. Therefore, the baculovirus vectors have attracted attention as a potential gene therapy vector.

Baculovirus vector has extraordinary properties as the gene transfer vector into human from the following reasons: 1) the virus gene is as much as 130kbp, which allows insertion of large (<15kbp) foreign genes, 2) since the virus gene is not expressed in mammalian cells, it hardly causes cytotoxicity and harmful immune response is not induced, 3) recombinant virus can be prepared in short time, 4) there exists no neutralization antibody against baculovirus in human, etc.

Until now, it has been reported that in order to improve the infection efficiency, an attempt has been made to provide other virus envelope proteins such as vesicular stomatitis virus G protein together with baculovirus (e.g. refer to Non-patent document 5) (FIG. 3 left). This remarkably improved the gene transfer efficiency into mammalian cells.

Further, another attempt has been made to replace baculovirus gp64 protein by other virus envelope proteins completely, and it is reported that even if gp64 protein is replaced by envelope proteins of closely related virus or vesicular stomatitis virus G protein, the recombinant virus can replicate in insect cells (e.g. refer to Non-patent document 6) (FIG. 3 right).

However, in these conventional methods, viruses are required to be infectious to insect cells to obtain high titer of stock virus. Thus in the methods by the recombination of virus DNA, only proteins having specific infectivity as envelope proteins could not be selected.

Therefore, it is impossible to make infection of these vectors specific and baculovirus vector capable of conferring specificity is not reported. Development of baculovirus vector which has the above-mentioned excellent properties and capable of conferring desired infectivity was strongly desired.

### (Non-patent document 1)

Matsuura, Y., Possee, R. D., Overton, H. A., and Bishop, D. H. L. (1987). Baculovirus expression vectors: the requirements for high level expression of proteins, including glycoproteins. J. Gen. Virol. 68,1233-1250.

### (Non-patent document 2)

Hofmann, C., Sandig, V., Jennings, G., Rudolph, M., Schlag, P., and Strauss, M. (1995). Efficient gene transfer into human hepatocytes by baculovirus vectors. Proc. Natl. Acad. Sci. USA 92, 10099-10103.

### (Non-patent document 3)

Boyce, F. M., and Bucher, N. L. R. (1996). Baculovirus-mediated gene transfer into mammalian cells. Proc. Natl. Acad. Sci. USA 93, 2348-2352.

### (Non-patent document 4)

Shoji I., Aizaki H., Tani H., Ishii K., Chiba T., Saito I., Miyamura T., and Matsuura Y. (1997). Efficient gene transfer into various mammalian cells including non-hepatic cells by baculovirus vectors. J. Gen. Virol. 78, 2657-2664.

### (Non-patent document 5)

Tani, H., Nishijima, M., Ushijima, H., Miyamura, T., and Matsuura, Y. (2001). Characterization of cell-surface determinants important for baculovirus infection. Virology 279, 343-353.

### (Non-patent document 6)

Mangor J.T., Monsam S.A., Johnson C.M., and Blissard G.W. (2001). A gp64-null baculovirus pseudotyped with vesicular stomatitis virus G protein. J. Virol. 75, 2544-2556.

### Disclosure of the Invention

An object of the present invention is to solve the above-mentioned problems and to achieve the following objects. Specifically, an object of the present invention is to provide a novel baculovirus vector can provide a desired protein not requiring infectivity to insect cells, on the surface of viral particle; method of producing thereof; and method of gene transfer using the baculovirus vector.

The inventors have reached the present invention based on the development of the method in which the gp64 gene of a baculovirus was made to be defective and instead the virus is allowed to uptake a protein of interest.

Specifically, the means for solving the problems of the present invention are as follows.
<1> A method of producing a baculovirus vector comprising; a process of cotransfecting at least a plasmid containing a gene coding a protein capable of being expressed on a cell surface and one of wild-type, mutated-type form, and recombinant baculovirus DNAs into insect cells, wherein a pseudotyped baculovirus which comprises at least one part of the baculovirus DNA and is coated with the protein capable of being expressed on a cell surface, is generated.
<2> A method of producing a baculovirus vector according to the <1>, wherein the baculovirus DNA is the recombinant baculovirus DNA and wherein, in the baculovirus DNA, a polyhedron gene and a first foreign gene are homologously recombined and a gp64 gene and a second foreign gene are homologously recombined.
<3> A method of producing a baculovirus vector according to the <1>, wherein the baculovirus DNA is the recombinant baculovirus DNA and wherein, in the baculovirus DNA, a polyhedron gene and a first foreign gene are homologously recombined.
<4> A method of producing a baculovirus vector according to the <3>, wherein, in the process of cotransfecting, a vector containing a second foreign gene is cotransfected at the same time, wherein a homologous recombination between a gp64 gene and the second foreign gene occurs, wherein a pseudotyped baculovirus which comprises the baculovirus DNA containing foreign genes and is coated with the protein capable of being expressed on a cell surface, is generated.
<5> A method of producing a baculovirus vector according to any one of the <2> to <4>, wherein the first foreign gene is at least one of a transgene and a marker gene.
<6> A method of producing a baculovirus vector according to any one of the <2> to <4>, wherein the second foreign gene is at least one of a transgene and a marker gene.
<7> A method of producing a baculovirus vector according to any one of the <1> to <6>, the protein capable of being expressed on a cell surface is a protein incapable of infecting insect cells.
<8> A method of producing a baculovirus vector according to any one of the <1> to <6>, the protein capable of being expressed on a cell surface is a protein capable of infecting insect cells.
<9> A method of producing a baculovirus vector according to <8>, further comprising a process of amplifying after the process of cotransfecting, wherein the process of amplifying comprises: infecting cells in which the plasmid containing a gene coding a protein capable of being expressed on a cell surface is expressed with a first pseudotyped baculovirus generated by the process of cotransfecting, and amplifying the virus to thereby generate a second pseudotyped baculovirus.
<10> A method of producing a baculovirus vector according to the <1>, wherein the baculovirus DNA is a recombinant baculovirus DNA which is defective in gp64 gene, and wherein, in the process of cotransfecting, a vector containing a transgene is further cotransfected.
<11> A method of producing a baculovirus vector comprising: at least a process of infecting insect cells expressing a protein capable of being expressed on a cell surface on its surface transiently with a gp64 gene defective baculovirus having a particle surface coated with gp64 protein, and allowing a viral particle coated with the protein capable of being expressed on a cell surface to bud from the cell surface.
<12> A baculovirus vector, which is produced by the method of producing a baculovirus vector of any one of the <1> to <11>.
<13> A baculovirus vector, comprising: a baculovirus DNA which is defective in gp64 gene, wherein the baculovirus is a pseudotyped virus coated with protein incapable of infecting insect cells.
<14> A baculovirus vector, comprising: a baculovirus DNA which is defective in gp64 gene, wherein the baculovirus is a pseudotyped virus coated with a protein capable of infecting insect cells, and wherein the protein is not a protein expressed from the baculovirus DNA.
<15> A baculovirus vector according to the <12> to <14>, which is capable of gene transfer specifically by selecting cells.
<16> A method of gene transfer comprising: transferring a gene into one of cells in vivo and cells in vitro using the baculovirus vector of any one of <12> to <15>.

### Brief Description of the Drawings

FIG. 1 is a diagram explaining an infection of baculovirus polyhedron into insect cells.
FIG. 2 is a diagram illustrating a life cycle of a baculovirus.

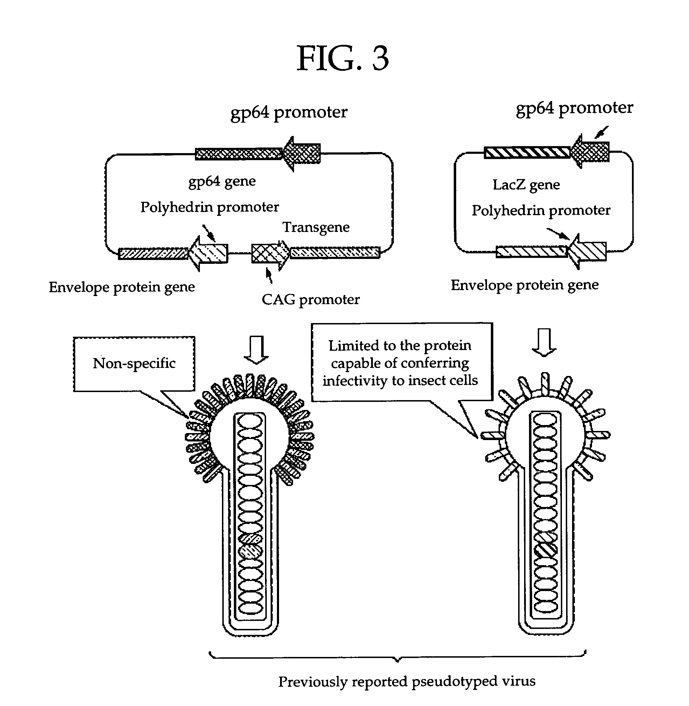

FIG. 3 is a diagram illustrating a conventional recombinant vector.
FIG. 4 is a diagram illustrating a conventional recombinant vector.
FIG. 5 is a diagram illustrating an example of the preparation of a novel pseudotyped baculovirus.
FIG. 6 is a diagram illustrating an example of the preparation of a novel pseudotyped baculovirus.
FIG. 7 is a diagram illustrating an example of the preparation of a novel pseudotyped baculovirus.
FIG. 8 is a diagram illustrating an example of the preparation of a gp64 knockout vector.
FIG. 9 is a diagram illustrating an example of the preparation of a gp64 defective baculovirus AcΔ64/GFP/LacZ.
FIG. 10 is a diagram illustrating an example of the preparation of a plasmid capable of expressing gp64 protein transiently in insect cells.
FIG. 11 is a diagram illustrating an example of the preparation of a gp64 gene defective virus AcΔ64/GFP/LacZ *64 coated with a gp64 protein transiently.
FIG. 12 is a diagram illustrating an example of amplification of AcΔ64/GFP/LacZ*64.
FIG. 13 is a diagram illustrating an example of the preparation of AcΔ64/GFP/LacZ *64 having a luciferase gene and coated with a gp64 protein transiently.
FIG. 14 is a diagram illustrating an example of the preparation of a pseudotyped virus coated with a vesicular stomatitis virus G protein.
FIG. 15 is a diagram illustrating an example of character of a pseudotyped virus coated with a vesicular stomatitis virus G protein.
FIG. 16 is a diagram illustrating an example of gene transfer by a pseudotyped virus coated with a vesicular stomatitis virus G protein.
FIGS. 17 is a diagram illustrating an example of a result of the neutralization test of a pseudotyped virus coated with a vesicular stomatitis virus G protein.

### Best Mode for Carrying Out the Invention

### Outline of construction of targeting vector

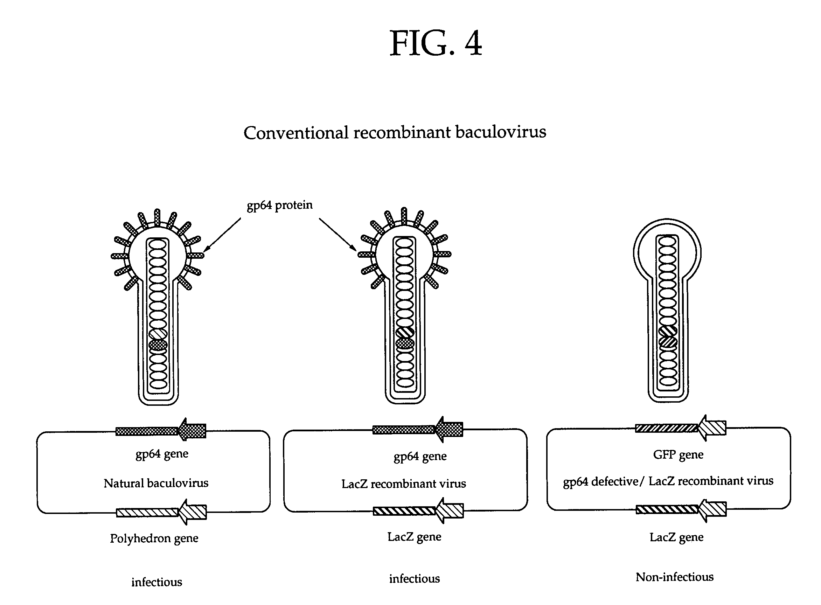

The present inventors have revealed that recognition of phosphatidylinositol (PI) on the cell surface by gp64 protein of baculoviral particle is important for the baculovirus to invade into mammalian cells (Tani et al., 2001). Since the PI is found in a variety of cells, it is impossible to confer cell specificity to a baculovirus vector as long as it retains gp64 (FIG. 4).

Further, as mentioned above, according to the method of recombining coat protein, it is essential for coat protein to retain infectivity to insect cells, kinds of coat proteins are restricted and only proteins having universal infectivity to cells could be selected.

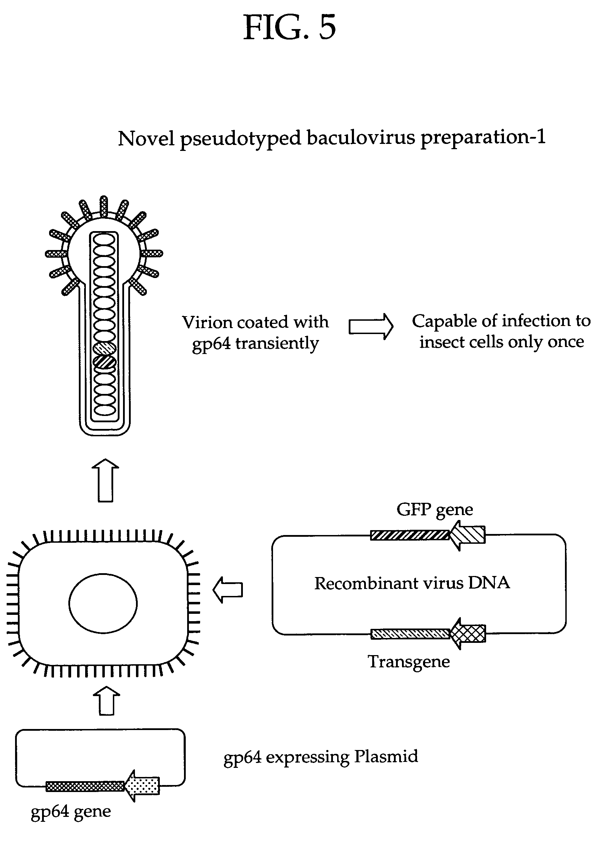

If the development of targeting vector is aimed for, it is required to make gp64 protein completely defective and prepare recombinant virus having only ligand molecule of interest. First, insect cells expressing a gp64 protein transiently is transfected with gp64 gene defective virus DNA to thereby construct a system from which viruses having a particle surface coated with gp64 protein transiently can be collected (FIG. 5).

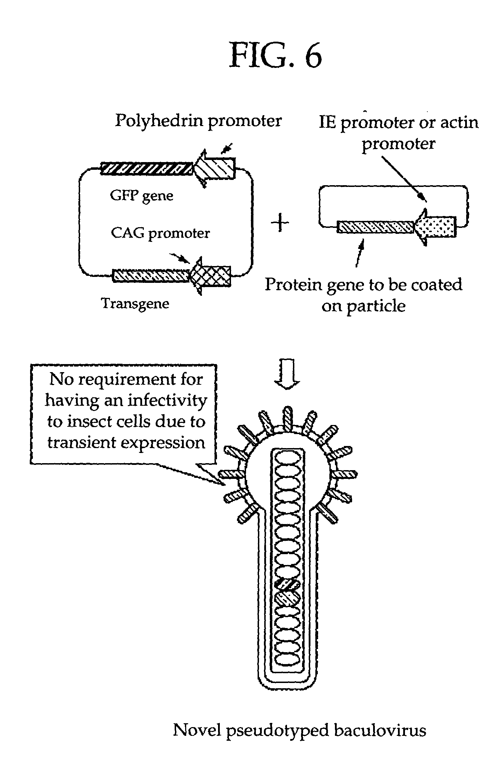

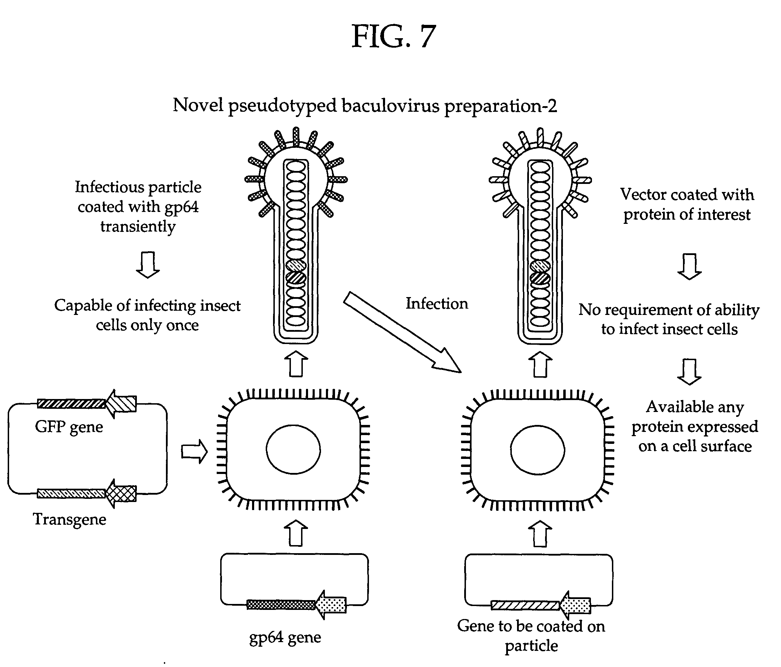

These recombinant viruses are capable of infecting insect cells via the gp64 protein only once. Thus, when this virus is allowed to infect the insect cells expressing ligand molecules of interest beforehand on the surface of the cell, viral particle coated with ligand molecule of interest buds from the cell surface (FIGS. 6 and 7). It was considered that allowing the virus to be coated with a protein transiently by expressing an envelope protein using a plasmid would be difficult from the viewpoint of the gene transfer efficiency.

The method of producing a baculovirus vector comprises a process of cotransfecting at least a plasmid containing a gene coding a protein capable of being expressed on a cell surface and one of wild-type, mutated-type form, and recombinant baculovirus DNAs into insect cells, wherein a pseudotyped baculovirus which comprises at least one part of the baculovirus DNA and is coated with the protein capable of being expressed on a cell surface, is generated.

In the present invention, a pseudotyped baculovirus refers to a baculovirus having a protein not derived from the virus DNA thereof on the virus surface. Specifically, it refers to a virus coated with the protein which is transiently expressed by the plasmid introduced separately. A baculovirus coated with gp64, a baculovirus envelope protein, is included in the pseudotyped baculovirus in case the gp64 protein is not derived from the virus DNA but from a plasmid, for example, for the reason that the virus DNA gp64 region of the baculovirus is defective. Here, "transiently expressed" refers to expression without being integrated into virus DNA.

The protein capable of being expressed on a cell surface may be a protein incapable of infecting insect cells or protein capable of infecting insect cells. Here, the protein capable of infecting insect cells refers to a protein, when it is expressed on a baculovirus envelope, enabling virus entry into insect cells. When this vector is used for transfection to human, etc. as it is, infectivity to insect cells is not required; however, when this vector is used as an intermediate for preparation of a final vector, the vector is required to be capable of infecting insect cells because it is allowed to propagate by infection to insect cells.

Examples of the protein capable of infecting insect cells include a gp64 protein, envelope proteins of closely related virus to baculovirus, and vesicular stomatitis virus G protein.

The protein incapable of infecting insect cells is not particularly limited as long as it is a protein capable of being expressed on a cell surface, but is preferably a ligand or receptor corresponding to receptor and antigen which are specific to the cell or tissue in vivo into which genes are expected to be transferred. Examples include molecules capable of recognize a cancer specific antigen and cell surface molecules different from normal cells, receptor molecules against the antigen expressed specifically in virus-infected cells such as AIDS virus.

Herein, the insect cells to be transfected are not particularly limited, but Sf9 cells can be suitably used.

The plasmid containing a gene coding a protein capable of being expressed on a cell surface can be prepared by suitably selecting a plasmid from known plasmids and inserting a gene coding a desired protein. Examples include pIB/gp64, pA3Fb/gp64 of the Examples described below, etc.

Moreover, the promoter in the plasmid is one other than a gp64 promoter, is preferably a promoter which expresses in insect cells and is particularly preferably an actin promoter.

The Baculovirus DNA may be any one of wild-type, mutated-type form, and recombinant baculovirus DNAs, but it is preferred that baculovirus DNA is recombinant baculovirus DNA where a homologous recombination between polyhedron gene and the first foreign gene is performed and a homologous recombination between gp64 gene and the second foreign gene is performed.

Further, it is preferred that baculovirus DNA is the recombinant baculovirus DNA where a polyhedron gene and a first foreign gene are homologously recombined. In this case, it is preferable that in the cotransfecting process, a vector containing the second foreign gene is cotransfected at the same time and homologous recombination between gp64 gene and the second foreign gene.

The foreign gene to be homologously recombined is preferably one of a transgene which is a gene of interest introduced into target cells and marker gene which is used as an index of isolation or introduction, etc. and more preferably, the first foreign gene is a transgene. Furthermore, the foreign gene to be homologously recombined preferably has a promoter which allows the gene to be expressed at high level in insect cells such as a polyhedrin promoter. When a final vector adapted to host is prepared, a promoter which expresses in the cell is used as the promoter of the transgene.

The promoter expressed in human is not particularly limited, but a CAG promoter composed of chicken β actin promoter and CMV enhancer, CMV promoter, RSV promoter, etc. are preferred.

Further, the foreign gene to be introduced is not particularly limited as long as it can be expressed by the above-mentioned promoter, but from the viewpoint of usefulness, defective genes related to various kinds of genetic diseases, cytokines, neurotrophic factors, nonself-antigen gene, nucleotide sequence coding virus antigen, etc., cancer suppressor gene, antisense sequence such as Ras, cancer gene, or suicide gene such as thymidine kinase, etc. is preferred.

Further, it is preferable that the method of producing a baculovirus vector further comprises an amplifying process after the cotransfecting process, the amplifying process comprises infecting cells in which the plasmid containing a gene coding a protein capable of being expressed on a cell surface is expressed with a first pseudotyped baculovirus generated by the process of cotransfecting, and amplifying the virus to thereby generate a second pseudotyped baculovirus from a viewpoint that amplification is easily achieved.

Further, the method of producing a baculovirus vector according to another aspect of the present invention comprises a cotransfecting process in which a plasmid containing a gene coding a protein capable of being expressed on a cell surface, a recombinant baculovirus DNA which is defective in gp64 gene, and a vector containing a transgene is cotransfected into insect cells and a pseudotyped baculovirus coated with the protein capable of being expressed on a cell surface, is generated. The recombinant baculovirus DNA which is defective in gp64 gene is preferably a recombinant baculovirus DNA which has two kinds of genes containing a polyhedrin promoter and a marker downstream thereof.

Further, the method of producing a baculovirus vector according to another aspect of the present invention comprises at least a budding process, the process comprising infecting insect cells expressing a protein capable of being expressed on a cell surface on its surface transiently with a gp64 gene defective baculovirus having a particle surface coated with gp64 protein, and allowing a viral particle coated with the protein capable of being expressed on a cell surface to bud from the cell surface.

Here, insect cells expressing a protein capable of being expressed on a cell surface on its surface transiently refers to insect cells expressing protein by a plasmid, which can be obtained by introducing a plasmid coding the protein.

The method of producing a baculovirus vector may further comprise a process for preparing gp64 a gene defective baculovirus having a particle surface coated with the gp64 protein before the above-mentioned process, i.e., the process for allowing a baculovirus having a particle surface coated only one generation with a gp64 protein to bud by transfecting insect cells expressing protein transiently with the gp64 gene defective virus DNA.

The baculovirus vector of the present invention is produced by the method of producing a baculovirus vector of the present invention.

Regarding other methods, etc. such as handling of insect cells, a general method of gene recombination and cotransfection, a similar technique as the known method of preparing a recombinant virus of insect cells (Yoshiharu Matsuura, Protein, Nucleic Acid and Enzyme, 37, 211-222, 1992, Yoshiharu Matsuura, baculovirus expression system, "gene transfer and expression/analysis method" edited by Takashi Yokota and Kenichi Arai, Yodosya, Yoshiharu Matsuura, Cell 33 (2) 30-34, 2001).

For example, a transfer vector where a gene to be expressed is integrated, infectious virus DNA and, in case of the present invention, in addition, a plasmid are introduced into Sf9 cells (0.8 × 10⁶ / 35 mm dish) at the same time and cultured supernatant at 4th day is diluted to allow plaque formation. It is suspended in a 400 ml of culture medium, viruses are eluted from agar by vortexing and centrifuged to harvest the supernatant. This supernatant is inoculated into Sf9 cells, etc. expressing envelope protein transiently, and after cultivation, supernatant is collected once again to obtain white virus pellet. Plasmid is preferably introduced into Sf9 cells (1 × 10⁶ / 35 mm) using 2 µg of plasmid DNA and about 6 µl of transfection reagent (manufactured by UniFactor, Bridge, etc.).

Moreover, the baculovirus is infectious to mammalian cells and introduces gene into nucleus, but does not propagate in the cells. Therefore, in order to obtain high expression, it is required that virus having high infectious titer (1 × 10⁹-10 pfu / ml is prepared by purification and high copy gene is introduced. In the method of producing a baculovirus of the present invention, this can be achieved by infecting cells expressing transiently the plasmid having desired protein with gp64 gene defective baculovirus having a particle surface coated with gp64 protein, and carried out, for example, using the following known method except for making the plasmid expressed.

0.5 ml to 1.0 ml of stock virus (normally 1×10⁷⁻⁸ pfu / ml) is inoculated into Sf9 cells (1.0 × 10⁷ / 10 cm dish), after 4 days of infection, cultured supernatant is collected, lightly centrifuged (6, 000 g, 10 minutes) to thereby remove cell debris and stored. This surpernatant is centrifuged with 25,000 rpm in an SW28 rotor (Beckman) for 60 minutes at 4°C and the obtained virus pellet is suspended in 1 ml of PBS. This is loaded on 10 to 60% of sucrose gradients and is centrifuged with 25,000 rpm in an SW41 rotor (Beckman) and for 60 minutes at 4°C in an SW41 rotor (Beckman). The virus band is collected. After suspension in PBS, virus is centrifuged with 25,000 rpm in an SW41 rotor for 60 minutes at 4°C. The purified virus pellet is suspended in 1 ml of PBS and stored at 4°C with light shielded. The infectious titer of the purifed virus can be measured by plaque assay using Sf9 cells. Normally, 0.2 × 10¹⁰ pfu / ml of purified virus is obtained from 250 to 500 ml of cultured supernatant.

Moreover, the baculovirus vector according to another aspect of the present invention comprises a baculovirus DNA which is defective in gp64 gene, wherein the baculovirus is a pseudotyped virus coated with a protein incapable of infecting insect cells. Examples thereof include baculovirus vector which comprises a baculovirus DNA in which a marker gene with a polyhedrin promoter is introduced into the gp64 gene region containing a gp64 promoter region and polyhedron gene region, respectively, and which baculovirus vector is coated with any plasmid-derived protein capable of being expressed transiently on a cell surface and incapable of infecting insect cells, but the derivation of the protein incapable of infecting insect cells is not limited to these examples.

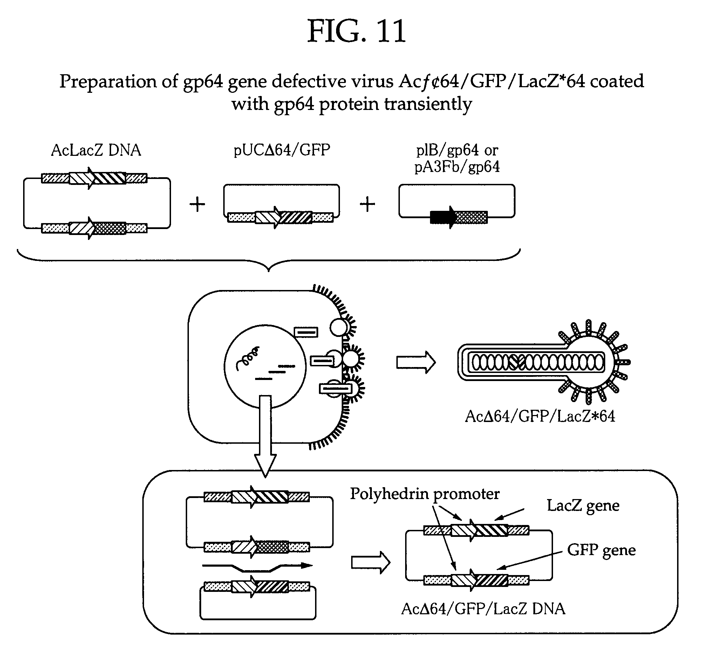

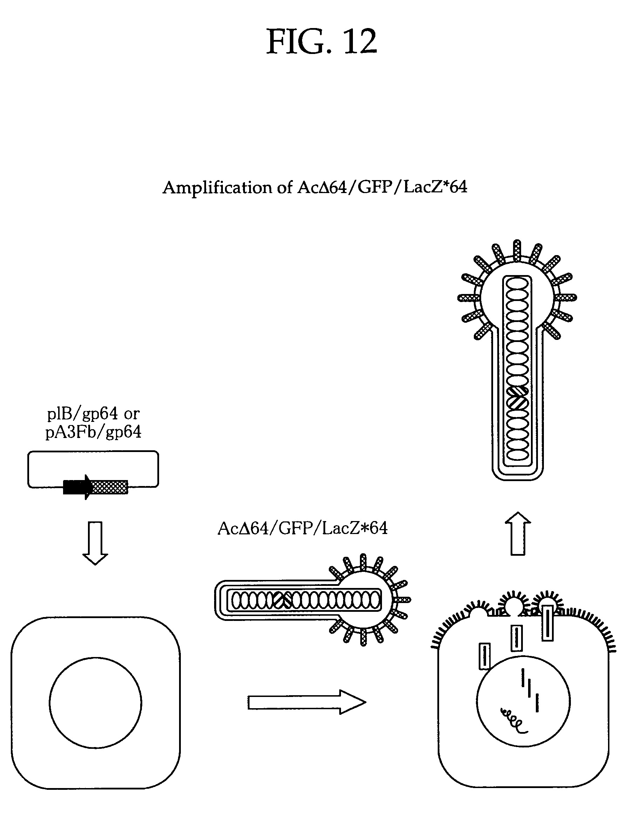

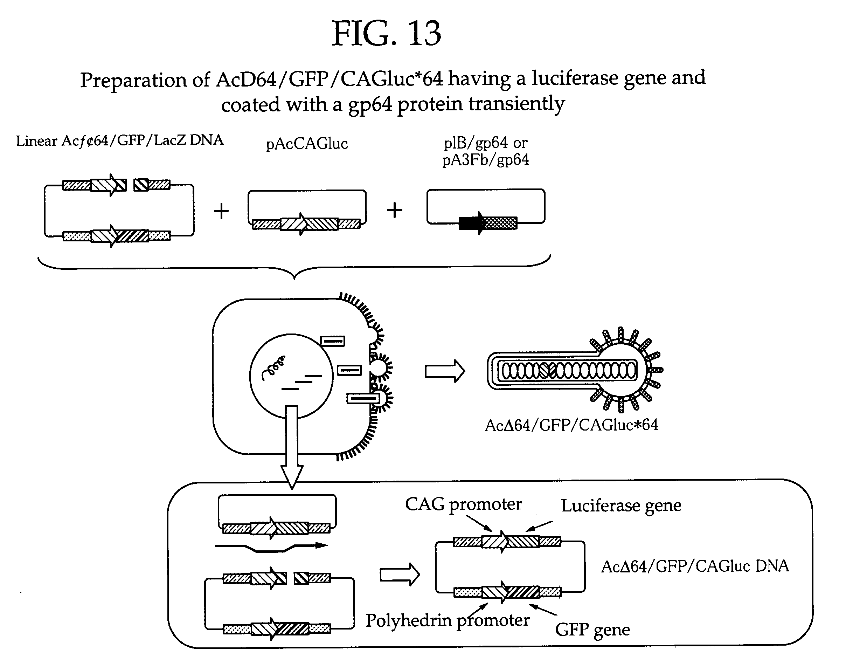

Moreover, another baculovirus vector of the present invention is a baculovirus vector comprising a baculovirus DNA which is defective in gp64 gene, wherein the baculovirus is a pseudotyped virus coated with a protein capable of infecting insect cells, and wherein the protein is not a protein expressed from the baculovirus DNA. The baculovirus vector may be a vector not containing transgene and coated transiently with gp64 protein such as AcΔ64/GFP/LaCZ*64 (FIGS. 11 and 12) mentioned later, a vector with transgene integrated and the vector being coated transiently with gp64 protein such as AcΔ64/GFP/CAGluc*64 (FIG. 13), and a vector with transgene integrated and the vector being coated transiently with protein capable of being expressed on a cell surface other than gp64 protein and capable of infecting insect cells.

The pseudotyped baculovirus vector coated with protein capable of infecting insect cells can be used for propagation of the baculovirus vector in insect cells and used as a stock.

Moreover, the pseudotyped baculovirus vector coated with protein incapable of infecting insect cells can be used as a final vector for introducing gene into either cells in vivo including human or cells in vitro. When the baculovirus vector is administerd into living organism, it can be administered orally or non-orally (e.g. injection into vein, muscle, peritoneal cavity, and under or into the skin, intrarectal administration, transmucosal administration, administer into an organ, etc.). The amount and number of doses can be suitably controlled according on the weight of the subject and gene to be transferred.

In some cases, gene expression in vivo is interfered with inactivation by a complement, but the inactivation can be circumvented by applying a nafamstat mesilate which is a proteolysis inhibitor of complement at the same time.

The method of gene transfer of the present invention comprises transferring a gene into one of cells in vivo and cells in vitro using the baculovirus vector of the present invention.

Gene transfer into mammalian cells can be carried out in the same way as that of normal virus vector. For example, mammalian cells into which the gene is transferred is prepared in a plate or dish with 60 to 80% of mammalian cells confluent. The method of infecting is the same as that in normal virus inoculation. First, cultured supernatant is removed, recombinant virus is inoculated at a multiplicity of infection (moi) of 10 to 100 and incubated for 30 to 60 minutes. Then, medium is added and cultured at 37°C. At 24 to 48 hours postinfection, cells are collected and gene expression is examined. Further, since baculovirus may be inactivated by a compliment in serum, it is preferred that the fetal bovine serum in culture medium is inactivated so as not to work.

### Examples

The baculovirus vector and method of producing thereof, and method of transferring gene using the baculovirus vector of the present invention will be illustrated in further detail with reference to several examples below, which are not intended to limit the scope of the present invention.

### (Example 1)

### Preparation of gp64 knockout vector

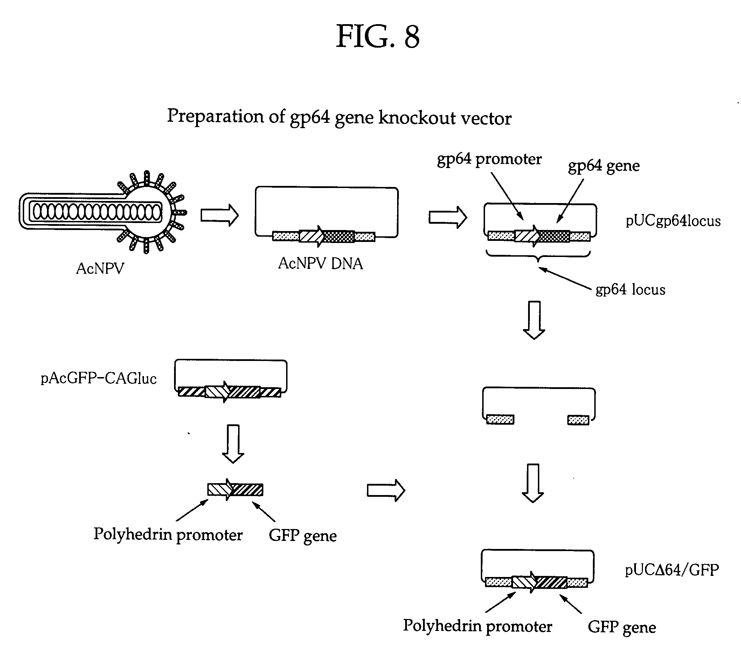

In order to eliminate the expression of gp64 by replacing gp64 gene region of baculovirus with polyhedrin promoter and green fluorescent protein (GFP) gene, knockout vector pUCΔ64/GFP was prepared in the procedure shown in FIG. 8.

An EcoRI-SmaI fragment (nt 107,325-112,049) containing the gp64 gene region of baculovirus (AcNPV) was inserted into the same restriction enzyme site of pUC18 to create pUCgp64locus. Next, transfer vector pAcGFP-CAGluc (Tani et al., 2001) where GFP gene and luciferase gene were inserted downstream of polyhedron promoter and downstream of CAG promoter (Niwa et al., 1986) developed by Miyazaki et al., respectively, was cleaved at EcoRV and SnaBI and fragment containing polyhedrin promoter and GFP gene was collected. The fragment was inserted into the blunted site with klenow fragment after cleavage of pUCgp64locus at SpeI (nt 109,761) and BglII (nt 108,039) to thereby prepare gp64 gene knockout vector, pUCΔ64/GFP.

### (Example 2)

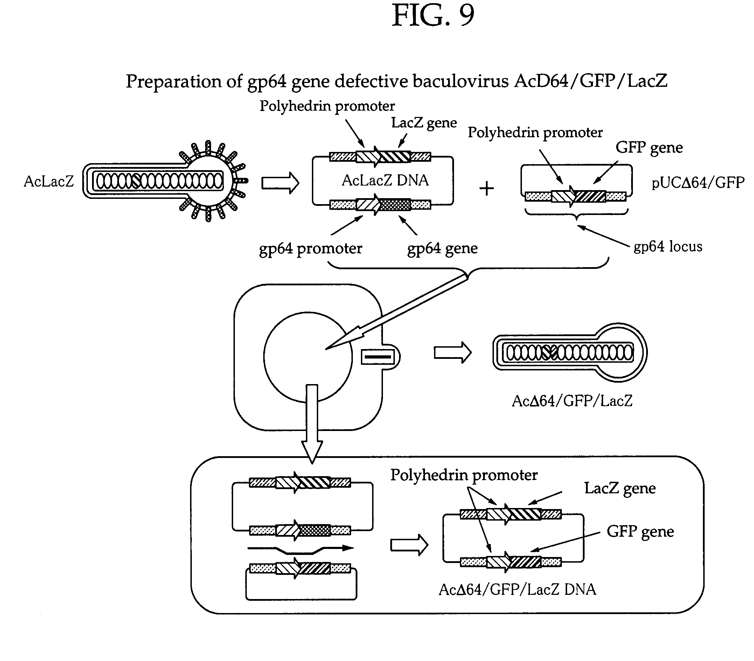

### Preparation of gp64 gene defective baculovirus AcΔ64/GFP/LacZ (FIG. 9)

An infectious virus DNA of recombinant baculovirus (AcLacZ) where beta galactosidase (LacZ) gene downstream of the polyhedrin promoter is inserted was extracted, with which Sf9 cells were cotransfected together with the gp64 gene knockout vector, pUCΔ64/GFP. In the nucleus of the insect cells, homologous recombination between virus DNA and pUCΔ64/GFP occurred, thus resulting in recombinant virus AcΔ64/GFP/LacZ where gp64 gene region of baculovirus DNA is replaced by polyhedrin promoter and GFP gene. This virus is defective in gp64 gene and it's promoter region. Thus, virus is defective in envelope protein completely and shows no infectivity. Thus, AcΔ64/GFP/LacZ is modified so that it can infect only once by coating the AcΔ64/GFP/LacZ with gp64 protein transiently.

### (Example 3)

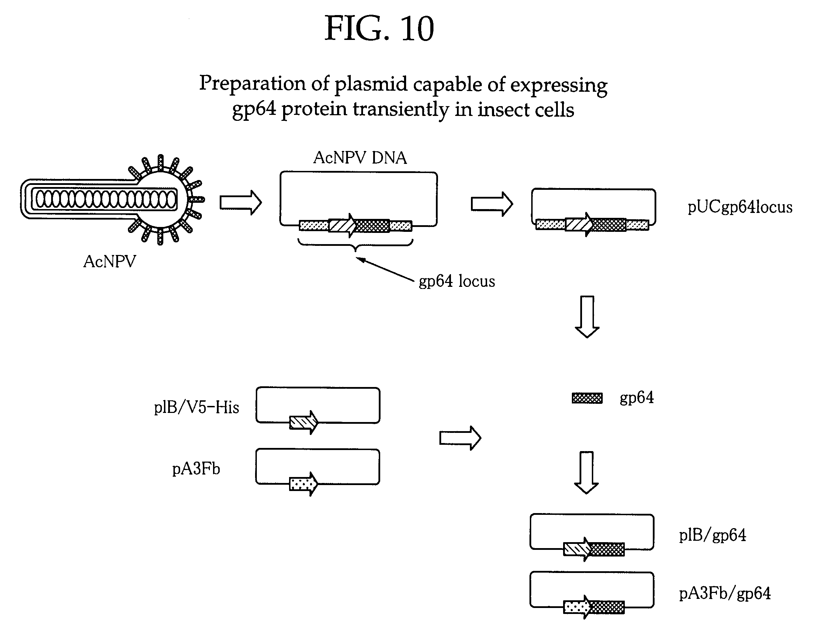

### Preparation of plasmid capable of expressing gp64 protein transiently in insect cells (FIG. 10)

In order to prepare a plasmid capable of expressing gp64 protein in Sf9 cells, a SpeI-BglII fragment containing gp64 gene was cut out from pUCgp64locus and the fragment was inserted into the pUC18 which was cleaved with the same restriction enzyme to prepare pUCgp64. Moreover, a Hind III-EcoRI fragment containing gp64 gene was cut out from pUCgp64 and the fragment was inserted into the pIB/V5-His (Invitrogen) which was cleaved with the same restriction enzyme to prepare expression vector pIB/gp64. Genes inserted downstream of baculovirus early gene promoter (IE promoter) of pIB/V5-His can be expressed efficiently in insect cells. Moreover, a similar expression vector was prepared utilizing an actin promoter which is transcribed at high rate in insect cells. First, gp64 gene was amplified by PCR using pUCgp64locus as a template. As a primer, gp64-Fw (Bgl): AAAGATCTACCatggtaagcgctattgttt (sequence number 1) and gp64-Rv (Sal): TTGTCGACttaatattgtctattacggttt (sequence number 2) were used. The amplified gp64 gene was cleaved with BgIII and SalI and inserted into the BglII-SalI site of pA3Fb to prepare a pA3Fb/gp64.

### (Example 4)

### Preparation of gp64 gene defective virus AcΔ64/GFP/LacZ*64 coated with gp64 protein transiently (FIG. 11)

Sf9 cells were cotransfected with the above-mentioned infectious virus DNA of AcLacZ, pUCΔ64/GFP, and pIB/gp64 or pA3Fb/gp64. This causes occurrence of homologous recombination between virus DNA and pUCΔ64/GFP in the nucleus of the insect cells, thus resulting in recombinant virus AcΔ64/GFP/LacZ where gp64 gene region of baculovirus DNA is replaced by polyhedrin promoter and GFP gene. Since this virus is defective in gp64 gene and it's promoter region, the virus cannot propagate in normal insect cells. However, a gp64 protein is supplied on the surface of the cells in trans by cotransfection with pIB/gp64 or pA3Fb/gp64, and thus, the virus can bud coated with gp64 protein and keeping infectivity in spite of defective in gp64 gene. In addition, the recombinant virus can be distinguished from the parent AcLacZ using the expression of GFP as an index. Specifically, the recombinant virus expresses both LacZ and GFP. Practically the virus was separated as follows. Specifically, from the cultured supernatant on the 4th day after transfection, both GFP- and LacZ-positive plaques where homologous recombination was considered to occur were collected by normal plaque assay using Sf9 cells cotransfected with pIB/gp64 or pA3Fb/gp64 the day before. Plaque assays were repeated four times to be purified, and thus, obtained AcΔ64/GFP/LacZ*64 where gp64 gene region was replaced by a polyhedrin promoter and GFP gene and coated with a gp64 protein transiently to retain infectivity.

### (Example 5)

### Amplification of AcΔ64/GFP/LacZ*64 (FIG. 12)

The above-isolated AcΔ64/GFP/LacZ*64 is coated with gp64 protein transiently; however, gp64 is deleted, so it can infect normal Sf9 cells only once and cannot be amplified. However, virus with high titer can be easily prepared by infecting Sf9 cells which is transfected with pIB/gp64 or pA3Fb/gp64 beforehand and gp64 is expressed on it's surface.

### (Example 6)

### Preparation of recombinant virus expressing various kinds of reporter gene

In order to allow the transfer gene to be expressed in target mammalian cells to which gene to be transferred, transfer vectors, pAcCAGluc, pAcCAGDsRed and pAcCAGGFP where three kinds of reporter genes, a firefly luciferase gene, red fluorescent protein (DsRed) gene, GFP gene, respectively, was inserted downstream of the CAG promoter were used. These vectors are designed so that a homologous recombination occurs in the polyhedron gene region. The preparation of the pAcCAGluc has already been reported (Tani et al., 2001). pDsRed2-N1 (Clontech) was cleaved with KpnI, DsRed gene was collected and inserted into the pAcCAGMCS2 (Shoji et al., 1997) cleaved with the same restriction enzyme to prepare pAcCAGDsRed. pIRES2-EGFP (Clontech) was cleaved with NcoI, a fragment containing GFP was blunt with klenow fragment, and then inserted into cleaved with SmaI and NotI and bunt-ended pAcCAGMCS2 with klenow fragment to prepare pAcCAGGFP.

FIG. 13 shows a preparation procedure of recombinant virus using pAcCAGluc. DNA was extracted from AcΔ64/GFP/LacZ*64 and was cleaved at the unique Bsu36I site present in LacZ gene to thereby convert the virus DNA into linear. Sf9 cells were cotransfected with this linear virus DNA, pAcCAGluc, and pIB/gp64 or pA3Fb/gp64. And GFP-positive and LacZ-negative plaques were collected in a similar way. Plaque assays were repeated four times to be purified and recombinant baculovirus AcΔ64/GFP/CAGluc*64 having luciferase gene was prepared. By infecting Sf9 cells expressing gp64 protein which were transfected with pIB/gp64 or pA3Fb/gp64 beforehand, stock viruses having a high titer were prepared.

### (Example 7)

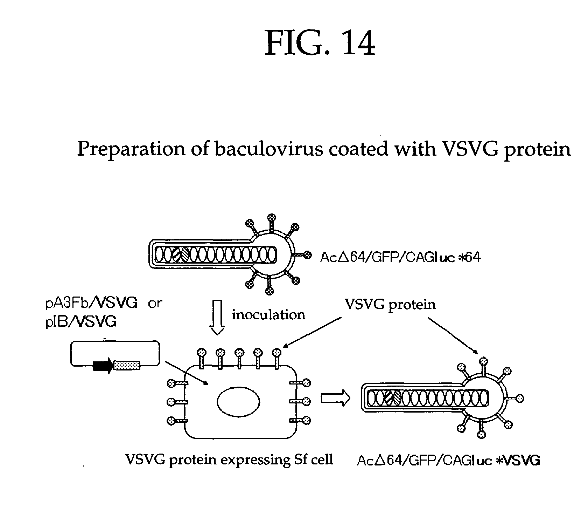

### Preparation of pseudotyped virus coated with vesicular stomatitis virus G protein (FIG. 14)

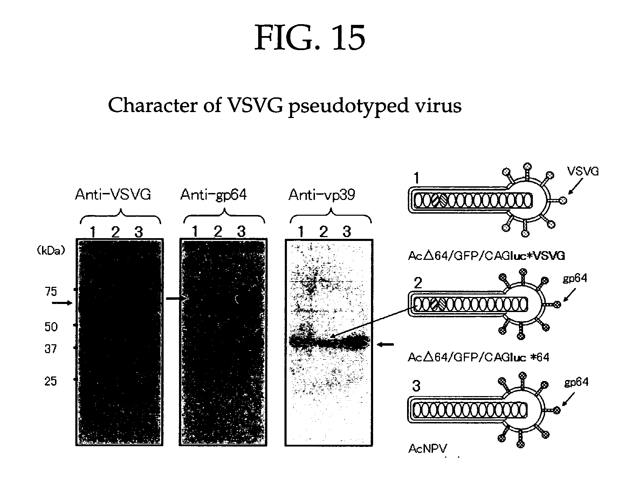

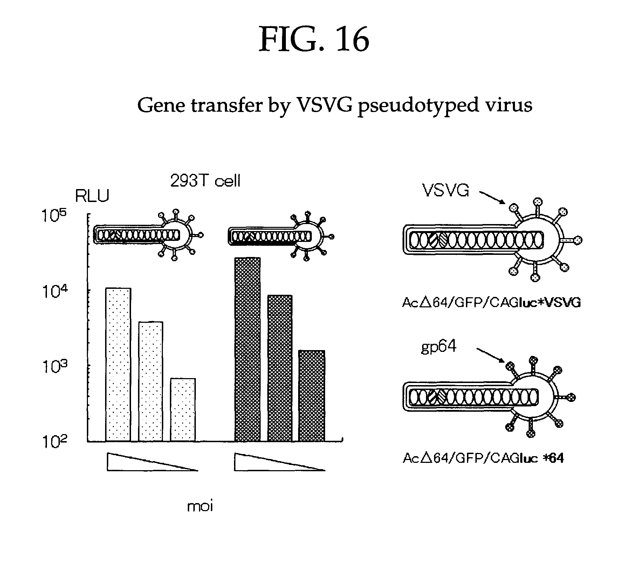

As an example of transgene, G protein gene of vesicular stomatitis virus was examined. pIB/VSVG and pA3Fb/VSVG where G protein gene of vesicular stomatitis virus was inserted into pIB/V5-His and pA3Fb, respectively, were prepared. One of these plasmids was transfected into Sf9 cells and after 24 hours, Sf9 cells was infected with AcΔ64/GFP/CAGluc*64 to thereby prepare pseudotyped virus AcΔ64/GFP/CAGluc*VSVG coated with vesicular stomatitis virus G protein instead of gp64 protein. It was confirmed that these viruses were defective in gp64 protein and kept vesicular stomatitis virus G protein by immunoblotting of purified virus using anti-gp64 antibody and anti-vesicular stomatitis virus antibody (FIG. 15). Further, it was found that the pseudotyped virus AcΔ64/GFP/CAGluc*VSVG coated with vesicular stomatitis virus G protein transiently infected a wide variety of mammalian cells efficiently like the pseudotyped virus AcΔ64/GFP/CAGluc*64 coated with gp64 protein transiently. FIG. 16 shows the results of inoculation of AcΔ64/GFP/CAGluc*64 and AcΔ64/GFP/CAGluc*VSVG, respectively, into 293T cells (obtained from ATCC) at an moi of 10, 30 and 50. Cultured supernatant was removed, virus was inoculated, after 60 minutes absorption, medium was added and incubated at 37°C. At 24 to 48 hours postinfection, cells were collected and luciferase gene expression was measured by Bright-Glo luciferase Assay system (manufactured by Promega) and the measurement value is shown.

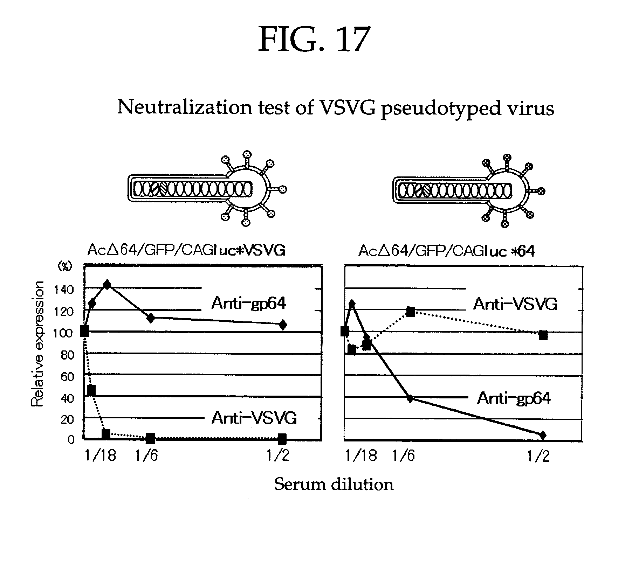

Further, a neutralization test was performed by neutralizing the AcΔ64/GFP/CAGluc*64 and AcΔ64/GFP/CAGluc*VSVG before cell inoculation using specific antibodies against gp64 and vesicular stomatitis virus G protein (VSVG), respectively (Anti-gp64: anti-gp64 antibody clone #AcV1 (Virology 125, p432-444 (1983)), Anti-VSVG : anti-VSVG antibody) and by infecting 293T cells. AcΔ64/GFP/CAGluc*VSVG was neutralized well by specific antibody against VSVG (FIG. 17). The neutralization test was performed as follows: the virus vector and antibody were mixed in a dilution concentration shown in horizontal axis of the figure, after 1-hour pre-incubation at room temperature, 293T cells was infected and expression was measured. In horizontal axis of the figure, the value of 1/2 represents the concentration where virus vector to antibody is 1: 1. Thus it was found that according to the method of producing a baculovirus of the present invention, without using infectivity of vesicular stomatitis virus G protein to insect cells, baculovirus vector coated with the protein were propagated to obtain. Therefore, it was demonstrated that desired envelope protein was free to be coated regardless of its infection to insect cells. Moreover, according to this method, after infection to the cell to which gene is transferred, the envelope protein is not expressed again and highly safe baculovirus can be prepared.

### (Example 8)

### Preparation of baculovirus expressing two proteins on it's particle surface: baculovirus having two envelope protein which determine host specificity of measles virus

Measles is an acute viral infectious disease, which shows strong infectivity and kills a million people mainly in developing countries every year. Measles virus has two, H and F, envelope proteins on the particle surface and the H protein decides tissue specificity of infection. Specifically, it is reveled that while measles virus strain (Edmonston strain) passaged in laboratory can utilize CD46 or SLAM (Signaling lymphocyte activating molecule; CDw150) as a receptor, a strain freshly isolated from outside utilize SLAM alone as a receptor. Recombinant baculoviruses expressing H and F proteins on it's particle surface derived from Edmonston strain or freshly isolated strain, respectively, were prepared and whether the infection specificity of measles virus can be reproduced in baculoviruses was examined.

A plasmid where H and F gene of measles virus derived from Edmonston strain or freshly isolated strain were integrated instead of pA3Fb/VSVG in Example 7, together with AcΔ64/GFP/CAGluc, was cotransfected to prepare AcΔ64/GFP/CAGluc*MV-H/F. Next, infection specificity of these baculoviruses were evaluated with hamster derived cell line (CHO cell) expressing SLAM or CD46. As a result, it was confirmed that the baculovirus vector coated with H and F proteins derived from Edmonston strain infected CHO cell expressing SLAM and CHO cell expressing CD46. In contrast, the baculovirus coated with H and F proteins derived from freshly isolated strain was capable of gene transfer only into CHO cell expressing SLAM. Positive control virus, AcΔ64/GFP/CAGluc*VSVG, infected both of CHO cells and negative control virus, AcΔ64/GFP/CAGluc, which is defective in gp64 protein showed no infectivity.

This result indicates that two virus proteins can be provided with biological activity retained on the particle surface of baculovirus. It is considered that by providing two proteins on the particle surface, more accurate targeting can be achieved. Moreover, not only providing virus envelope protein, alternatively, by providing virus receptor molecule or single-chain antibody against cancer antigen on the particle surface, suicide genes such as thymidine kinase can be introduced only into cells expressing envelope protein due to infection with virus or cancer cells and, by using prodrug in combination, only target cells can be excluded from living body. Especially, AIDS virus infects chronically or persistently, expresses envelope protein highly on the surface of the infected cells, and thus, it is expected that by recombinant baculovirus provided with receptor and co-receptor, only infected cells are excluded from living body.

According to the present invention, the problems in conventional technologies can be solved and a novel baculovirus vector which can provide a desired protein not requiring infectivity to insect cells, on the viral particle surface; method of producing thereof; and method of gene transfer using the baculovirus vector, can be provided.

## Claims

1. A method of producing a baculovirus vector comprising: a process of
cotransfecting at least a plasmid containing a gene coding a protein capable of being expressed on a cell surface and one of wild-type, mutated-type form, and recombinant baculovirus DNAs into insect cells,
wherein a pseudotyped baculovirus which comprises at least one part of the baculovirus DNA and is coated with the protein capable of being expressed on a cell surface, is generated.

2. A method of producing a baculovirus vector according to claim 1, wherein the baculovirus DNA is the recombinant baculovirus DNA and
wherein, in the baculovirus DNA, a polyhedron gene and a first foreign gene are homologously recombined and a gp64 gene and a second foreign gene are homologously recombined.

3. A method of producing a baculovirus vector according to claim 1, wherein the baculovirus DNA is the recombinant baculovirus DNA and
wherein, in the baculovirus DNA, a polyhedron gene and a first foreign gene are homologously recombined.

4. A method of producing a baculovirus vector according to claim 3, wherein, in the process of cotransfecting, a vector containing a second foreign gene is cotransfected at the same time,
wherein a homologous recombination between a gp64 gene and the second foreign gene occurs, and
wherein a pseudotyped baculovirus which comprises the baculovirus DNA containing foreign genes and is coated with the protein capable of being expressed on a cell surface, is generated.

5. A method of producing a baculovirus vector according to any one of claims 2 to 4, wherein the first foreign gene is at least one of a transgene and a marker gene.

6. A method of producing a baculovirus vector according to any one of claims 2 to 4, wherein the second foreign gene is at least one of a transgene and a marker gene.

7. A method of producing a baculovirus vector according to any one of claims 1 to 6, the protein capable of being expressed on a cell surface is a protein incapable of infecting insect cells.

8. A method of producing a baculovirus vector according to any one of claims 1 to 6, the protein capable of being expressed on a cell surface is a protein capable of infecting insect cells.

9. A method of producing a baculovirus vector according to claim 8, further comprising a process of amplifying after the process of cotransfecting,
wherein the process of amplifying comprises:
infecting cells in which the plasmid containing a gene coding a protein capable of being expressed on a cell surface is expressed with a first pseudotyped baculovirus generated by the process of cotransfecting; and
amplifying the virus to thereby generate a second pseudotyped baculovirus.

10. A method of producing a baculovirus vector according to claim 1, wherein the baculovirus DNA is a recombinant baculovirus DNA which is defective in gp64 gene, and
wherein, in the process of cotransfecting, a vector containing a transgene is further cotransfected.

11. A method of producing a baculovirus vector comprising:
at least a process of
infecting insect cells expressing a protein capable of being expressed on a cell surface on its surface transiently with a gp64 gene defective baculovirus having a particle surface coated with gp64 protein, and
allowing a viral particle coated with the protein capable of being expressed on a cell surface to bud from the cell surface.

12. A baculovirus vector, which is produced by the method of producing a baculovirus vector of any one of claims 1 to 11.

13. A baculovirus vector, comprising:
a baculovirus DNA which is defective in gp64 gene,
wherein the baculovirus is a pseudotyped virus coated with protein incapable of infecting insect cells.

14. A baculovirus vector, comprising:
a baculovirus DNA which is defective in gp64 gene,
wherein the baculovirus is a pseudotyped virus coated with a protein capable of infecting insect cells, and
wherein the protein is not a protein expressed from the baculovirus DNA.

15. A baculovirus vector according to any one of claims 12 to 14, which is capable of gene transfer specifically by selecting cells.

16. A method of gene transfer comprising:
transferring a gene into one of cells in vivo excluding human and cells in vitro using the baculovirus vector of any one of claims 12 to 15.
